(19) Europäisches Patentamt
European Patent Office
Office européen des brevets

(11) **EP 2 859 934 A1**

(12) **EUROPEAN PATENT APPLICATION**

(43) Date of publication:
**15.04.2015 Bulletin 2015/16**

(51) Int Cl.:
*B01D 53/00* [(2006.01)]    *B01D 53/72* [(2006.01)]
*B01D 53/26* [(2006.01)]    *C12M 1/00* [(2006.01)]

(21) Application number: **14184552.9**

(22) Date of filing: **12.09.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.10.2013 IT PN20130058**

(71) Applicant: **Parker Hannifin Manufacturing S.r.l.**
**20094 Corsico (MI) (IT)**

(72) Inventor: **Favero, Chiara**
**35010 Perarolo di Vigonza (PD) (IT)**

(74) Representative: **Gonella, Mario et al**
**Propria S.r.l.**
**Via della Colonna, 35**
**33170 Pordenone (IT)**

(54) **Improved device for biogas processing**

(57)    A device for processing a non-compressed gas stream, particularly biogas, consisting of a heat exchanger, a liquid separator particularly for water, a blower to blow and transport said gas stream to said first heat exchanger and said separator, and a filter operating as a coalescence filter for filtering out volatile impurities and substances and/or substances in the form of aerosols.

Said filter must operate at a temperature between 0 and 10°C and is located downstream of said liquid separator.

Said first heat exchanger, said liquid separator, and said filter are preferably contained in a single common enclosure and are integrated into the same functional structure and placed immediately adjacent to each other.

The device operates continuously by controlled, automatic "oscillation" of the temperature of the cooling device (chiller) connected to the heat exchanger, in which said "oscillation" is dependent and controlled by continuously measuring and checking temperature T1 at the outlet of first heat exchanger 1 compared to a preset value.

FIG. 8

**EP 2 859 934 A1**

## Description

The present invention refers to an improved device equipped with means achieving highly efficient purification and filtration of a gas containing a methane fraction, a carbon dioxide fraction, and a mixture of other substances, present specifically in the form of aerosols.

The preferred sector for use of the present invention is in the processing of biogases produced in various ways, which are already known, for the purpose of making them usable for common uses, particularly so that said biogas, after said processing, can be put into a methane gas distribution network, typically for general or civilian use, or for direct use as a fuel in combustion engines in order to produce electrical power.

The biogas generated at production facilities and/or plants in general contains a considerable quality of various substances other than methane and carbon dioxide, which need to be removed from the biogas flow provided to the end users because they are hazardous or highly polluting and/or harmful.

Indeed, as soon as biogas is produced at the source, it typically contains a proportion of moisture together with a mixture of oils, particularly limonene, toluene, cumene, and styrene, as well as other substances such as siloxanes.

Bear in mind that the definition of "end use" actually includes two very different uses:

- the first use consists of the biogas being used as a fuel in a combustion engine; in this case the carbon dioxide is released into the atmosphere after combustion;
- the second use consists of the gas flow being fed into a combustible gas distribution network, generally for civilian use. In this case, before the biogas is fed into said network, it is subjected to one or more types of processing to remove the fractions that absolutely must not be injected into the gas distribution network.

While the moisture is removed by known means and processes, extensive filtering techniques are used to remove and/or neutralize the other substances in the biogas, by having the biogas pass through columns containing activated charcoal or equivalent substances.

However, this procedure is very costly given that the activated charcoal needs to be replaced quite frequently along with other products, because of their continual use.

This process turns out to be prohibitively expensive either because of the direct cost of the materials being replaced or because of the indirect, yet equally high, costs of plant shutdowns, labor, and disposal or regeneration of the spent materials.

And even with this processing and these precautions, if the biogas thus provided is sent to combustion engines as a fuel, there will be a heavy economic burden of having to replace those engines very frequently due to their delicate nature, since their service life lasts about one year, after which they become must less efficient.

However it is used, biogas must still first be subjected to one or more filtration processes and moisture removal, and, in addition, a filtration process to filter out the oil components contained in the biogas from the source which are caused by the same type of material or substances at the source of the biogas fermentation or production process.

The solutions currently being implemented must also take into account the fact that, unlike the filtration/purification treatments for compressed gas, especially air, the way biogas is currently processed, it is supplied at a pressure comparable to atmospheric pressure or even at a slightly lower pressure.

This leads to the requirement that pressure losses in processing facilities generally need to be very low and certainly much lower than in conventional facilities that treat compressed gases.

Patent RU2118560 discloses a method for processing biogas that is essentially based cooling the biogas by having it pass through a cold water bath.

This causes separation of the various types of gases, which are supplied at different pressures depending on the processing temperature.

However, this method is mainly intended for the separation of various types of gases and not for the removal of non-gaseous contaminant substances, particularly oils, which are present in the initial biogas.

Patent EP 1,149,805 A1 discloses a method for processing fruit and plant products that are only available during a particular season and which need to be subjected to anaerobic fermentation.

In certain cases, especially when citrus rinds and associated plant waste products need to be processed, some substances are produced in the plant masses, especially certain types of oils that need to be removed from the process at the very beginning.

However, this process teaches us that these substances need to be removed before anaerobic fermentation and consequently, if additional substances containing even minimal quantities of these oils and/or siloxanes (a class of chemical compounds known per se, characterized by the presence of silicon atoms bonded in various ways to oxygen atoms) and/or sulfuric acid (H2S) are produced during the subsequent anaerobic fermentation phase, they must be intercepted and removed.

It is therefore desirable to make an improved device for processing biogas for the purpose of intercepting and removing nearly all foreign substances present in the biogas, particularly certain oils, certain types of siloxanes, and possibly sulfuric acid; this is the main purpose of the present invention.

This purpose is achieved by a device made according to the enclosed claims.

The features and advantages of the invention will become clear from the following description, given as a non-limiting example, in reference to the enclosed drawings in which:

- figure 1 shows a schematic diagram of a first embodiment of a device according to the invention,
- figure 2 shows a schematic diagram of a second embodiment of a device according to the invention,
- figure 3 shows a schematic diagram of a third embodiment of a device according to the invention,
- figure 4 shows a schematic diagram of a further improved embodiment comprising a combination of the improvements of the foregoing figures 2 and 3,

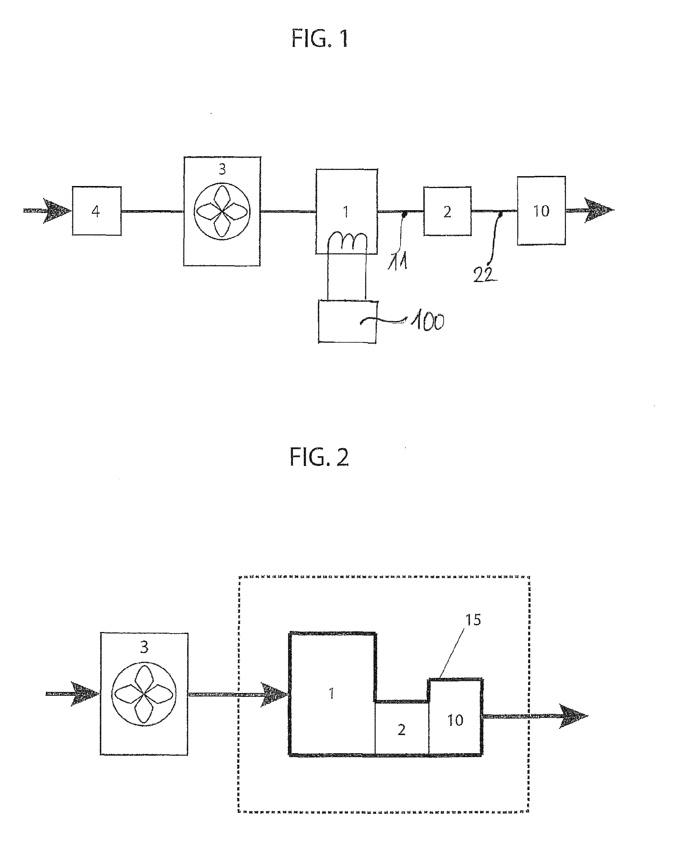

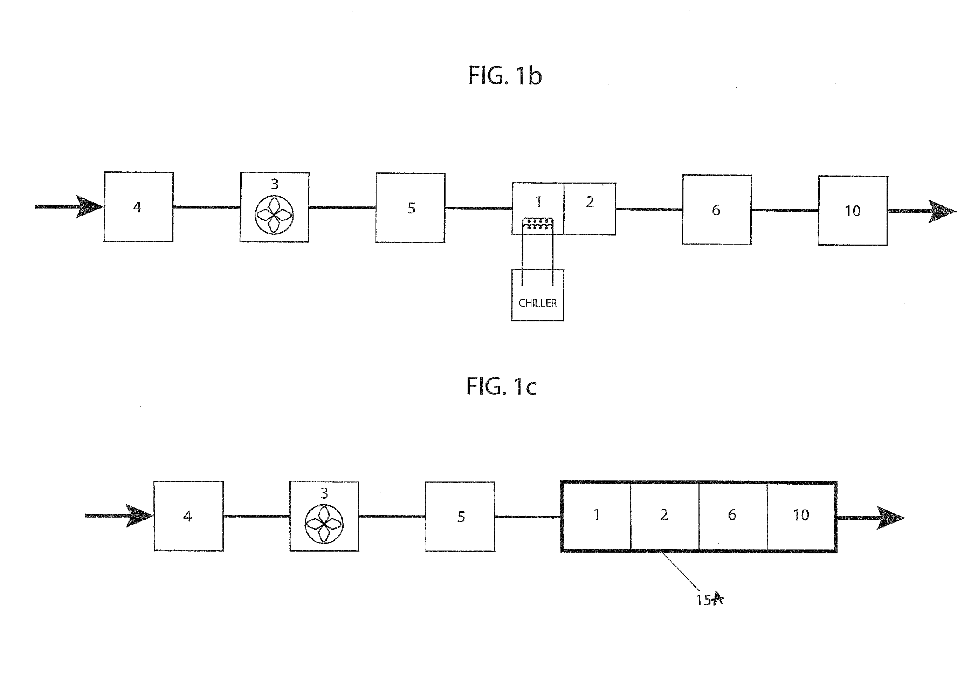

- figure 1b shows an improved variant of the device shown in figure 1,

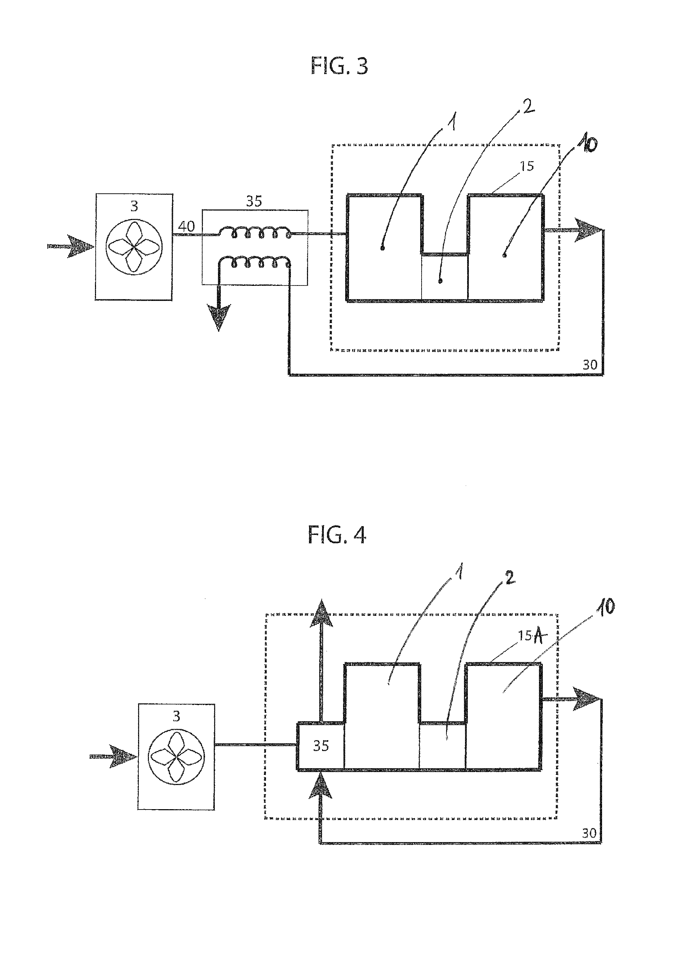

- figure 1c shows an embodiment of the device in figure 1 made using the improvement suggested in figures 3 and 4,

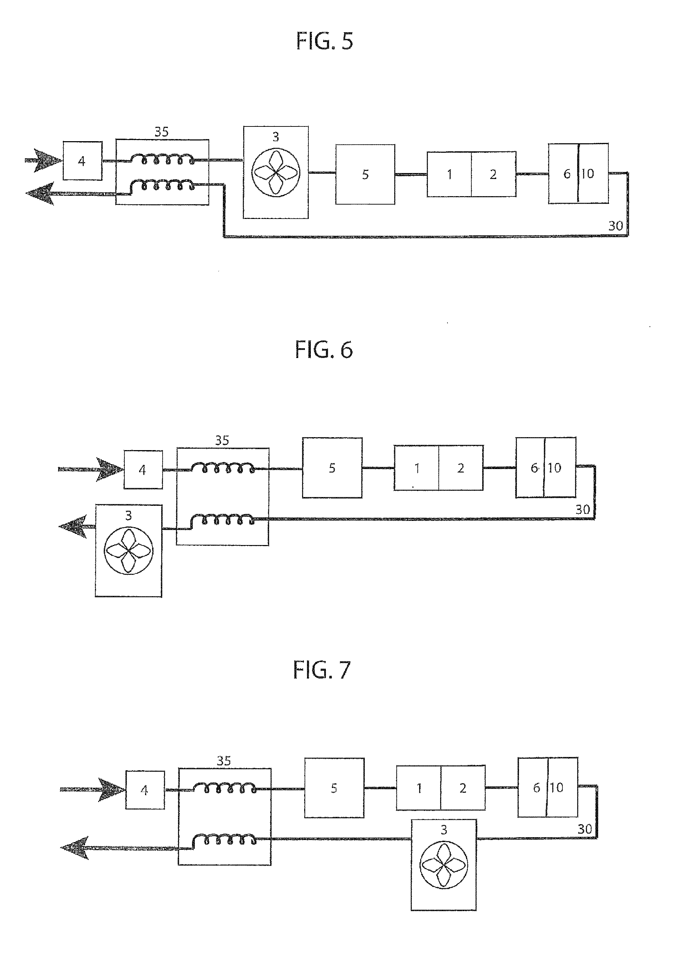

- figures 5, 6, and 7 show schematic diagrams of various embodiments of the device shown in a general form in figure 1 c,

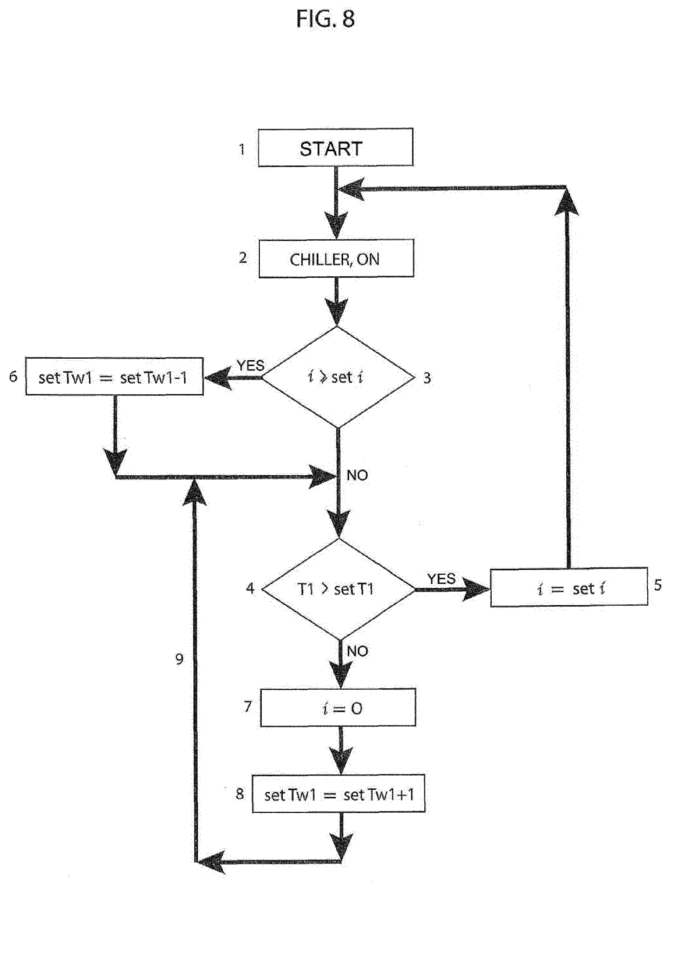

- figure 8 contains a mid-length cross-section view of a component in the device of the invention,

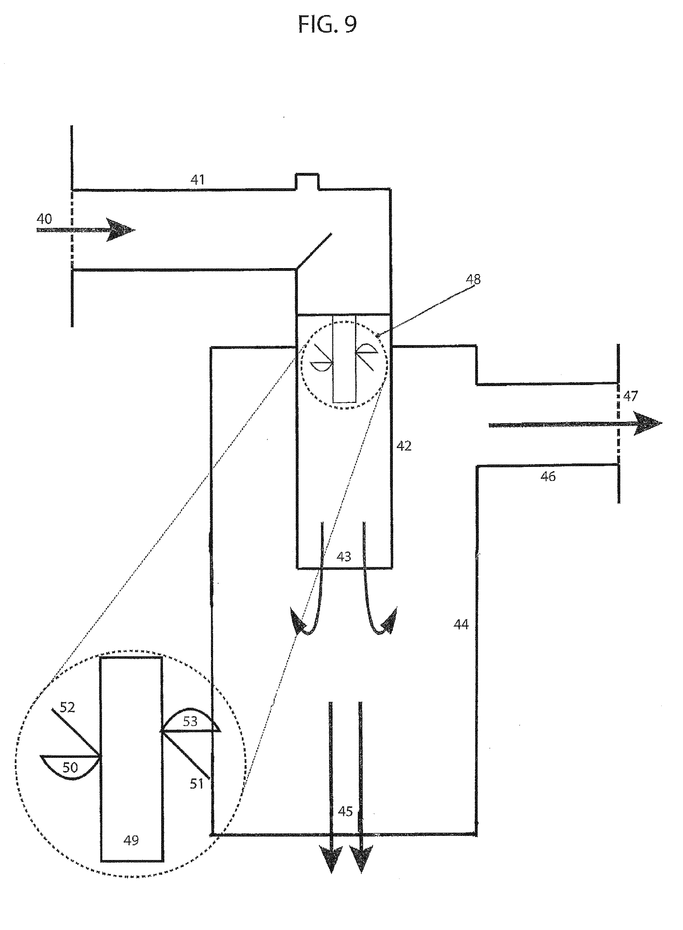

- figure 9 shows a flow chart for the operation of the control and monitoring means of a device according to the invention.

The invention described hereinafter is essentially based on a discovery arrived at by observing various applications and devices involving specific dehumidification and purification conditions and requirements for various types of gas, said discovery suggesting and leading to a solution which, in part, transfers some solutions from one application to another application, with modification and optimization.

In concrete terms, it has been observed that some generally contaminating elements in compressed air coming from industrial compressors are, to some degree, the same as or very similar to the contaminating substances in biogas, such as various oils that prevent direct use either as methane gas in a distribution network or as a direct supply to combustion engines.

However, it is not possible to mechanically transfer compressed air filtration technologies to the field of biogas filtration for the simple reason that, although the contaminating substances are similar, they are not the same, and so compressed air filters only work optimally for compressed air, since the coalescence effect during compressed air processing is still effective at ambient temperature, at which said filters are used.

Consequently, it has been observed that if these filters are used at very low temperatures close to 0°C--a limit which obviously cannot be crossed if the biogas has not been dried first--they yield excellent filtering results, including for said contaminating elements present in the biogas, which is the phenomenon on which the present invention is based.

Although this is already known in the field, let us recall that, as a general rule, the lower the temperature of the medium or gas being filtered, the better the operation of the filter.

This condition is reiterated here explicitly because it is the essential condition on which the device of the invention is made and operates.

In reference to figures 1 and 1b, a device for processing biogas for the purpose of removing the substances mentioned above essentially consists of:

- a first stage which includes a first heat exchanger 1 equipped with a first pipe through which said gas passes; said heat exchanger is connected by known means to chiller 100 (this is merely a sample configuration; said chiller can naturally be replaced advantageously with any cold water source) which is capable of creating a low-temperature fluid flow through the first branch of said first heat exchanger 1, which operates as an evaporator;
- a second stage located downstream of said heat exchanger, and which includes a liquid separator 2, particularly for water, generally consisting of a separation device, an improvement of which will be shown in detail in the description which follows.
- a final filter 10 located downstream of condensation separator 2 for intercepting the smallest remnants of oil (of various sources and types, either plant or animal) present in the biogas, which are stopped by a type of filter that generally takes advantage of the "coalescence" phenomenon, which is especially effective here because, as just mentioned, the temperature of the biogas leaving filters 2 and 6 is still substantially low and therefore suitable for effective filtration.

The feature of this final filter element 10, which makes it particularly useful and effective, is a result of three specific conditions, i.e.:

- its position in the biogas stream,
- in combination with its type,
- and furthermore in combination with its operating conditions.

Indeed, said final filter element 10 is implemented advantageously by a filter which operates according to the coalescence principle and is generally made according to patent DE 19520504 A1, which is especially well suited to intercepting aerosols, which behave as coalescing substances.

In addition, as shown in figure 1, said filter 10 is placed downstream of said liquid separator 2 so that a portion of the contaminating substances is intercepted by said separator 2 they reach filter 10.

This second condition protects the filter because it results in a much smaller quantity of said foreign substances passing through it, leading to less frequent replacement of the filter and therefore a first substantial economic benefit;

- means 3 normally referred to as a "blower" for blowing and conveying said gas stream, coming from a biogas source 4, through said first heat exchanger 1 and said liquid separator 2.

Said blower 3 is indispensable because, unlike compressed gases for industrial use, the gas is at a pressure of about one atmosphere, that is, about one bar, or it might even be at a slightly lower pressure.

Hence the need to provide said blower 3 to ensure that the biogas passes through said heat exchanger 1, said separator 2, and said filter 10.

Said first heat exchanger 1 for exchanging heat between gases in order to cool a gas--biogas in the present case--until the moisture in the gas condenses, for the purpose of removing it and ultimately drying the resulting gas, is a universally known technique which will therefore not be explained further; suffice it to mention patent (Italian Application) nos. PN2013000009 and PN2011A000019 by the same applicant, and the subsequent third-party patents cited in said patents by the applicant.

Liquid separator 2 is also a device known per se and widely used in industry; In general, this separator is based on screen elements for intercepting certain substances, such as moisture cooled to the condensation point, referred to as "demisters," or devices operating according to the centrifugal effect.

The third condition, which experimentation has shown to be of crucial importance, is the fact that said final filter 10 works at a relatively very low temperature of no more than 10°C and, in particular, its operation is optimized if it operates at a temperature not exceeding 5°C; this condition is very different from the normal operating conditions of this filter, since it has been observed that the ability of such a filter, as described, to filter out oils and other substances is enhanced if the filter operates at these relatively very low temperatures for a biogas.

To that end, in reference to figure 2, it is advantageous for said filter 10 to be cooled by the biogas passing through it, which obviously requires that the biogas enter final filter 10 at this temperature.

In order to meet this condition, it is especially useful for the biogas leaving heat exchanger 1 at a relatively low temperature compatible with the optimum operating temperature of said filter 10 not to be heated incidentally as it travels from said heat exchanger 1, through separator 2, and on to said filter 10.

In order to meet this condition, it is extremely advantageous to eliminate any possibility of environmental heating of the biogas before it reaches final filter 10. This requirement is met if said first heat exchanger 1, said liquid separator 2, and said final filter 10 are integrated according to a strict mechanical series and connections contained in a single enclosure 15, preferably with very effective insulation as shown schematically in figure 2, and especially if they are placed immediately adjacent to each other so that:

- outlet 11 and its corresponding pipe from said heat exchanger 1 are substantially the same as inlet pipe and the corresponding inlet of said liquid separator 2,
- and outlet 22 of said liquid separator 2 are substantially the same as the inlet pipe and the corresponding inlet of said filter element 10.

In essence, a single functional structure 15 is made, providing both total mechanical integration and optimum functional integration, as an expert in the field will have readily understood.

In addition, once again in reference to figure 2, since the blower located upstream of the device generally reduces its size and therefore its overall cost, and given that moving the biogas through the blower is one of the major costs of operating the facility, said blower 3 is advantageously located upstream of first heat exchanger 1.

Two advantageous embodiments of the above-described invention are presented in reference to figures 1b and 1c.

An improved embodiment of the assembly of a device according to the invention is shown in figure 1b; this improved embodiment introduces:

- pre-filter 5
- and post-filter 6.

These two filters are generally static filters serving different purposes; first filter 5 is located immediately upstream of first heat exchanger 1 for a first, substantial reduction of the dust present in the biogas, and second filter 6, which has a better filtering capability enhanced by the fact that the temperature of the biogas is much colder in this filter than when it enters first cycle 5, is placed immediately downstream of liquid separator 2 and upstream for final filtration of the finest dust from the biogas.

Consequently, said pre-filter 5 is advantageously placed upstream of said first heat exchanger 1 and post-filter 6 is placed between said condensation separator filter 2 and said final filter 10.

Figure 1c shows a schematic of the combination of the solution shown in figure 1b, i.e. with filters 5 and 6, and the improvement of figure 2, i.e. with insulated enclosure 15A containing heat exchanger 1, liquid separator 2, second fine filter 6, and final oil filter 10 all in the same thermally insulated space.

An additional advantageous embodiment of the invention is shown in figure 3: this embodiment is essentially the same as the device of figure 2, with the only difference being that the biogas outlet pipe, identified as item no. 30, leads to a second heat exchanger 35, to which main pipe 40 of the incoming biogas stream coming from blower 3 and going toward first heat exchanger 1 leads, as the usage pipe being subjected to the heat exchange.

In a facility for cooling and condensing moisture present in a gas, the method for implementing:

- a main heat exchanger in which the gas is cooled to a temperature to condense its moisture,
- and a secondary heat exchanger placed upstream of the main heat exchanger and supplied with the cooled gas leaving the main condenser, with the dual purpose of pre-cooling the gas going to the main heat exchanger, thus realizing a significant energy savings, or, alternatively, heating the outgoing gas if required and desirable, is known and widely used.

However, in the present application this solution provides an incremental benefit in that the outgoing temperature from filter 10 is already very low, less than 10°C as previosuly mentioned, and preferably even lower.

The relative humidity is 100%; consequently, if there is an external drop in temperature (for example, during winter months) it could result in condensation of water in the tubes. Post-reheating decreases the relative humidity and avoids this problem.

Having a relative humidity lower than 100% is sometimes a requirement of the engine. The gas-gas heat exchanger furthermore acts as an economizer by pre-cooling the incoming gas and reducing the amount of heat to be exchanged in heat exchanger 1 in order to reach T<10°C.

In order to make maximum use of the energy content in the outgoing biogas, said second heat exchanger 35, as shown in figure 4, is assembled in a way comparable to the way in which first heat exchanger 1, separator 2, and filter 10 were made.

In essence, said second heat exchanger 35 is made as an integrated unit mechanically adjacent to said devices 1, 2, and 10, and in particular it is placed immediately upstream of first heat exchanger 1 and therefore contained in insulated structure 15A

The solutions shown in figures 3 and 4 concerning second heat exchanger 35 show a typical way of positioning and including blower 3.

In addition, those same two figures 3 and 4 naturally give a schematic representation of configurations in which insulated structures 15 and 15A may simply be lacking; for the sole sake of simplicity, these same figures without said structures 15 and 15A have been omitted.

Furthermore, other positioning solutions for said blower 3 can be seen, as illustrated schematically in figures 5, 6, and 7.

Indeed, each said solution offers functional and design features that may be preferred by the user of the device from time to time, as desired, depending on the design, functional, and economic requirements.

For example, in figure 5 blower 3 is placed immediately downstream of second heat exchanger 35, which makes the blower operate at a lower temperature and therefore with the biogas slightly more dense, so the blower ultimately works more efficiently.

Figure 6 shows blower 3 placed downstream of the entire device, so the blower can operate at ambient temperature, but it is blowing completely purified gas, which improves the operating conditions of the blower and lengthens the time between maintenance operations.

Lastly, figure 7 shows blower 3 placed downstream of all the filters but immediately upstream of second heat exchanger 35. This solution allows the biogas to be blown when completely filtered, as in the foregoing solution, but also allows blower 3 to operate at a very low temperature of about 3°C, which may be advantageous under certain operating conditions, especially in a facility.

The solutions described above effectively solve the stated problem; however, the overall efficiency of the entire device, especially its filtering efficiency, is noticeably optimized if condensation separator 2 is made as shown in figure 9.

The condensation separator is made in a substantially known way with a first housing 44 that is typically cylindrical and vertical.

A second housing 42 fed by a horizontal pipe 41 through which the biogas enters, is placed vertically and coaxially in said filter; in practice, this second housing 42 acts as a pipe which feeds the gas flow to first housing 44.

Said second pipe ends with a lower opening 43 from which the biogas is expelled, and then the biogas enters the space between first housing 46 and second housing 44 by known means, and is then subjected to the action and effect of filtration, which intercepts the moisture and ultimately expels it out through said lower opening 45.

Lastly, the processed biogas is conveyed to the outside by outlet pipe 46, which is fed with the biogas in said space, and final outlet 47.

Very advantageously, the separation action is found to be substantially enhanced by component 48 with transversal blades suitably placed and angled inside the vertical section of second housing 42.

Said component 48 can be described as a set of blades comprising a plurality of separate blades 50, 51, 52, and 53 positioned radially and lying substantially in the same plane perpendicular to the travel axis of the biogas.

However, it must be clear that in this improvement said blades are immobile; indeed, it has been found experimentally that the presence and action of said blades lead to increased separation efficiency, since the blades cause an increase in the centrifugal effect which is precisely the effect that separates out the condensation.

It is indeed true that condensation separator filter 2 can also cause an undesirable pressure loss, but this drawback can easily be mitigated with an adequate geometry of the various components in the filter, and the benefits of a significant increase in the centrifugal effect decisively exceed the moderate drawback of the pressure loss incurred.

If a chiller or equivalent means of water cooling is used, the operation of this device can be triggered by known control and monitoring means that are appropriately programmed and connected.

The optimum operating mode of the device can advantageously be explained in detail as follows, in reference to figure 8 which shows an operation flow chart and graphically illustrates the corresponding algorithm.

BLOCK 1: The start of operation is initiated, with all circuits, sensors, functional components, and other items involved being activated and powered up;

BLOCK 2: Chiller 100 is activated, which activates operation of first heat exchanger 1,

BLOCK 3: this is an "IF" block; here, the following condition is checked:

$$i >= set\ i$$

The letter "i" represents the number of cycles counted by a cycle counting operator since the last reset of an appropriate register storing the number of cycles completed.

The cycles taken into account by the cycler counter operator are typically the chiller 100 operating cycles, but other parameters representing the operation of the device may also be used.

The value of "set i" is an arbitrary number of cycles that is entered externally and used as one of the parameters for monitoring the device.

BLOCK 4: this is an "IF" block; here, the following condition is checked:

$$T1 > set\ T1$$

where "T1" is the temperature at the outlet of heat exchanger 1 and "set T1" is the corresponding "set point" which can be entered arbitrarily and is used as one of the parameters for monitoring the device.

It must be added, however, that although the "T1" parameter introduced above refers to the value of the temperature at the outlet of first heat exchanger 1, this value can also refer to another parameter, the temperature of which is unequivocally correlated to the temperature of the gas at the outlet of said first heat exchanger; consequently, in the remainder of this description said "T1" parameter will continue to be cited as pertaining to the temperature of the biogas from heat exchanger 1, but it is explicitly understood that this can refer to any other temperature correlated to said temperature of the biogas at the outlet of heat exchanger 1.

BLOCK 5: this is the operation where the number of cycles counted by the cycle counter is "forced" to the pre-defined value set i.

The meaning of this operation will be explained extensively below.

BLOCK 6: Set Tw1 = set Tw1-1

This is the operation where the previously set temperature setting Set Tw1 of the cooling fluid in chiller 100 is reduced by a pre-defined amount and "forced" to assume a new value represented symbolically by:

$$Set\ Tw1-1;$$

BLOCK 7: the number of cycles counted by the cycle counter is reset to zero;

BLOCK 8: This is the operation where the previously set temperature setting Tw1 of the cooling fluid in chiller 100 is increased by a pre-defined amount and "forced" to assume a new value represented symbolically by:

$$Set\ Tw1+1.$$

The algorithm describe above, entered into control and monitoring means in the device and not expressly illustrated, causes the device of the invention to operate as follows:

to) when the device is turned on, after chiller 100 has been turned on (Block 2), control proceeds to block 3, which is an "IF" block; if the number of cycles "i" stored in the cycle counter register is NOT greater than or equal to the set point given by "set i", then control proceeds to block 4;

b) the condition T1 > set T1 is checked in this block 4; in practice, the objective is to check the behavior of the temperature at the outlet of first heat exchanger 1, since the lower this temperature (within obvious specific limits) the better the entire device operates, as seen earlier.

c) if this condition T1 > set T1 is confirmed, it means the device is operating at a higher temperature than a preset value; if so, control proceeds to block 5 in which the cycle counter register is forced to a preset number of cycles i = Set i; as we shall see in the subsequent block 3, which is activated during a subsequent phase, this condition is used to divert control to a subsequent phase for the purpose of reducing the temperature set point of chiller 100.

d) control returns to block 2, which turns on chiller 100 or confirms that it is already operating.

e) control returns to block 3; the condition previously set in block 5 is checked in this block and control obviously proceeds to block 6, since i set i is true (because of block 5).

f) in this block 6, the value of the temperature SET Point Tw1 (that is, the chiller temperature set point, not to be confused with Set T1, which is the temperature set point of the biogas) is reduced by a preset amount, assuming the value conventionally represented by Set Tw1-1 not to be significant per se in this description, since it becomes a design parameter that the designer can freely set.

In practice, the new temperature set point is lower than the previous one, which causes the desired condition of continued chiller operation.

g) in this phase control proceeds again to block 4, in which the phases previously described in detail in the foregoing phase c) are repeated; from this point forward, the sub-routine running through blocks 4, 5, 2, 3, 6, 4, 5, etc. is automatically performed iteratively.

h) during the foregoing phase c), block 4, if the following condition:

T1 > Set T1 is NOT confirmed, it means that the temperature of the biogas at the outlet of first heat exchanger 1 is cold enough, but it may also be too cold; if this is the case, control resets the operation of the device by proceeding to block 7, where the number of cycles in the corresponding cycle counter register is reset to zero with the following setting: i = 0.

i) control then proceeds to block 8, where the value of chiller temperature SET Point "Tw1" is increased by a preset amount, assuming the value conventionally represented by: Set Tw1 +1.

Here again, this value is not significant per se in this description, since it becomes a design parameter which the designer can freely determine.
In practice, the new temperature set point Set Tw+1 is higher than the previous one, which causes a natural shutdown of the chiller either immediately or after a certain time interval (or partial operation or, in general, an energy savings); however, this condition obviously cannot be permanent, since it would lead to excessive heating; consequently, this condition is continuously checked by continuously monitoring changes in the checked temperature, that is, temperature T1 at the outlet of first heat exchanger 1.

j) control then proceeds again to block 4 by logical link 9, where temperature T1 is again checked as described above starting with phase b), block 4.

In practice, as an expert in the field will have already imagined, operation of the device according to the above algorith consists of a controlled "oscillation" of the Set point temperature Set Tw1 of the chiller temperature between the conventional values Set Tw1-1 and Set Tw1+1 (which, as explained earlier, are not fixed values but rather decreasing or increasing values compared to the previous value), in which said oscillation is checked and controlled in block 4 by continuously monitoring temperature T1 at the outlet of first heat exchanger 1.

**Claims**

1. Apparatus for drying and filtering a flow of not-compressed gas, in particular biogas, and constituted by:

   - a first stage which comprises a first heat exchanger (1), a branch of which is connected to a means able of cooling (100);
   - a second stage arranged downstream said first heat exchanger, and comprising a liquid separating filter (2) and especially water,
   - means (3) for the blowing and the conveying of said gas flow through said first and second stage,

   **characterized in that** it comprises a final filtering means (10) working as a coalescent filter, able of filtering contaminants and volatile compounds, and or formed as vaporized substances.

2. Apparatus according to claim 1, **characterized in that** said first heat exchanger (1) and said liquid separating filter (2) are able of supplying a gas flow to said final filtering means (10) at a temperature lower that 10°C, and preferably lower that 5°C.

3. Apparatus according to claim 1 or 2, **characterized in that** said final filtering means (10) is arranged downstream said a liquid separating filter (2).

4. Apparatus according to any of the previous claims, **characterized in that** it comprises even a pre-filter (5) placed upstream said first heat exchanger (1), and a post-filter (6) placed downstream said a liquid separating filter (2) and upstream said final filtering means (10).

5. Apparatus according to claim 3 or 4, **characterized in that**:

   - said first heat exchanger (1), said liquid separating filter (2) and said final filtering means (10) are enclosed inside a common insulating casing (15, 15a),

- and they are integrated in a same operating frame and arranged closely contiguous to each other, so that:

- the outlet mouth (11) of said first heat exchanger (1) basically coincides with the inlet mouth of said liquid separating filter (2),

- and the outlet mouth (22) of said liquid separating filter (29 basically coincides with the inlet mouth of final filtering means (10).

6. Apparatus according to any of the previous claims, **characterized in that** said blowing means (3) is arranged upstream said first heath exchanger (1).

7. Apparatus according to claim 6, **characterized in that** a second heat exchanger (35), provided with two distinct conduits for the thermal exchange, is arranged between said blowing means (3) and said first heat exchanger (1), wherein:

> - a first conduit (40) conveys said gas from an outer source to the inlet of said first heat exchanger (1),
> - a second conduit (30) takes said gas from said final filtering means and conveys it into said second heat exchanger (35).

8. Apparatus according to any claim from 1 to 5, **characterized in that** upstream said pre-filter (5), a second heat exchanger (35) is arranged, which is passed by two distinct conduits apt to the thermal exchange, wherein:

> - a first conduit (40) conveys said gas from an outer source into the inlet of said second heat exchanger (1),
> - a second conduit (30) takes said gas at the outlet of said final filtering means (10) and channels it into said second heat exchanger (35),
> - and **in that** said blowing means (3) is arranged between said second heat exchanger (35) and said pre-filter (5).

9. Apparatus according to any claim from 1 to 5, **characterized in that** upstream said pre-filter (5), a second heat exchanger (35) is arranged, which is passed by two distinct conduits apt to the thermal exchange, wherein:

> - a first conduit (40) conveys said gas from an outer source into the inlet of said second heat exchanger (1),
> - a second conduit (30) takes said gas at the outlet of said final filtering means (10) and channels it into said second heat exchanger (35),
> - and **in that** said blowing means (3) is arranged downstream said second heat exchanger 835).

10. Apparatus according to any claim from 1 to 5, **characterized in that** upstream said pre-filter (5), a second heat exchanger (35) is arranged, which is passed by two distinct conduits apt to the thermal exchange, wherein:

> - a first conduit (40) conveys said gas from an outer source into the inlet of said second heat exchanger (1),
> - a second conduit (30) takes said gas at the outlet of said final filtering means (10) and channels it into said second heat exchanger (35),
> - and **in that** said blowing means (3) is arranged downstream said final filtering means 810) and upstream said second heat exchanger (35).

11. Apparatus according to any previous claim, **characterized in that** said liquid separating filter (2) is implemented by a device (41, 42, 43, 44, 45, 46), wherein the second casing (42) inside the first casing (44), is provided with an inner element (48) showing transversal blades (50, 51, 52, 53) which are firm with respect ton said casings (42, 44).

12. Apparatus according to any previous claim, **characterized in that** it operates according to an algorithm able of implementing the control of the gas temperature (T1) at the outlet of said first heat exchanger (1), or of a temperature therein correlated, with respect to a related SET POINT (SET T1), by measuring and maintaining of the temperature (Tw1) of the cooling fluid of said chiller (100), associated to said first heat exchanger (1) with regard to a respective defined SET POINT (SET Tw1).

13. Apparatus according to claim 12, **characterized in that**:

> - said Set point (SET T1) relevant to the temperature (T1) of the gas at the outlet of said first heat exchanger (1) can be selectively predetermined,
> - said Set Point (SET Tw1) of the cooling fluid of said chiller (100) is dynamically defined according to the difference between the gas temperature (T1) at the outlet of said first heat exchanger (1) and the respective SET POINT (SET T1).

FIG. 1

FIG. 2

FIG. 1b

FIG. 1c

FIG. 3

FIG. 4

## FIG. 5

## FIG. 6

## FIG. 7

## FIG. 8

FIG. 9

Europäisches
Patentamt

European
Patent Office

Office européen
des brevets

# EUROPEAN SEARCH REPORT

Application Number

EP 14 18 4552

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 900 408 A1 (INGERSOLL RAND ENERGY SYSTEMS [US]) 19 March 2008 (2008-03-19) * paragraphs [0010] - [0012], [0015] - [0018], [0021], [0023]; figures 1, 2 * | 1-3,6, 8-13 | INV. B01D53/00 B01D53/72 B01D53/26 C12M1/00 |
| X | WO 2010/020023 A1 (CAMILOTTI DANIEL [BR]; CAMILOTTI FLAVIO [BR]; CAMPOS JOAO [BR]) 25 February 2010 (2010-02-25) * page 3, line 21 - page 5, line 3; figure 1 * | 1,3,4,6 | |
| X Y | US 2013/209338 A1 (PRASAD ALAKH [CA] ET AL) 15 August 2013 (2013-08-15) * paragraphs [0030], [0031]; figures 1, 4, 5 * | 1-3,6,7, 12,13 11 | |
| X | US 2004/045440 A1 (BASEEN SANJIV K [US] ET AL BASSEEN SANJIV K [US] ET AL) 11 March 2004 (2004-03-11) * paragraphs [0035] - [0046]; figure * | 1-3,12, 13 | |
| Y | WO 2006/056831 A1 (ECOCIPREA S R L [IT]; GUALERZI NICO [IT]; BOMBARDI DANILO [IT]) 1 June 2006 (2006-06-01) * claims 1, 15; figure 1 * | 5 | TECHNICAL FIELDS SEARCHED (IPC) B01D C12M |
| Y | DE 37 12 209 A1 (HOELTER HEINZ [DE]) 27 October 1988 (1988-10-27) * column 1, line 62 - column 3, line 25; claim; figure * | 5 | |
| Y | DE 38 05 045 A1 (CASNICO MARINO [DE]) 31 August 1989 (1989-08-31) * figures 5, 7, 9, 10 * | 11 | |
| A | US 2007/289448 A1 (SILVA JOSEPH A [US] ET AL) 20 December 2007 (2007-12-20) * paragraph [0026]; figure 3 * | 1-13 | |

The present search report has been drawn up for all claims

| Place of search | Date of completion of the search | Examiner |
|---|---|---|
| The Hague | 4 February 2015 | Focante, Francesca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 18 4552

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2015

| Patent document cited in search report | | Publication date | Patent family member(s) | | Publication date |
|---|---|---|---|---|---|
| EP 1900408 | A1 | 19-03-2008 | CN<br>EP<br>US<br>US | 101185827 A<br>1900408 A1<br>2008066618 A1<br>2010107876 A1 | 28-05-2008<br>19-03-2008<br>20-03-2008<br>06-05-2010 |
| WO 2010020023 | A1 | 25-02-2010 | NONE | | |
| US 2013209338 | A1 | 15-08-2013 | CA<br>CN<br>EA<br>EP<br>JP<br>KR<br>US<br>WO | 2709722 A1<br>103079682 A<br>201390118 A1<br>2593211 A1<br>2013538880 A<br>20130097723 A<br>2013209338 A1<br>2012006729 A1 | 15-01-2012<br>01-05-2013<br>28-06-2013<br>22-05-2013<br>17-10-2013<br>03-09-2013<br>15-08-2013<br>19-01-2012 |
| US 2004045440 | A1 | 11-03-2004 | NONE | | |
| WO 2006056831 | A1 | 01-06-2006 | NONE | | |
| DE 3712209 | A1 | 27-10-1988 | NONE | | |
| DE 3805045 | A1 | 31-08-1989 | NONE | | |
| US 2007289448 | A1 | 20-12-2007 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2118560
- EP 1149805 A1
- DE 19520504 A1
- IT PN2013000009
- IT PN2011A000019